## Europäisches Patentamt
## European Patent Office
## Office européen des brevets

(19)

(11) Veröffentlichungsnummer: **0 163 042**
**B1**

(12) # EUROPÄISCHE PATENTSCHRIFT

(45) Veröffentlichungstag der Patentschrift:
06.04.88

(51) Int. Cl.⁴: **A 61 F  2/26**

(21) Anmeldenummer: **85103542.8**

(22) Anmeldetag: **15.06.82**

(54) **Femorale Hüftgelenkendoprothese.**

(30) Priorität: 30.06.81 DE 3125657
03.05.82 DE 3216533

(43) Veröffentlichungstag der Anmeldung:
**04.12.85 Patentblatt 85/49**

(45) Bekanntmachung des Hinweises auf die Patenterteilung:
**06.04.88 Patentblatt 88/14**

(84) Benannte Vertragsstaaten:
**AT BE CH DE FR GB IT LI NL SE**

(56) Entgegenhaltungen:
**DE-A-2 839 093
DE-A-2 931 750
FR-A-1 046 516
FR-A-1 416 534
US-A-3 228 393**

(73) Patentinhaber: **Waldemar Link GmbH & Co,
Barkhausenweg 10, D-2000 Hamburg 63 (DE)**

(72) Erfinder: **Link, Helmut D., Wildstieg 14, D-2000
Hamburg 65 (DE)**
Erfinder: **Keller, Arnold, An der Naherfurth 5,
D-2061 Kayhude (DE)**

(74) Vertreter: **Glawe, Delfs, Moll & Partner
Patentanwälte, Postfach 26 01 62 Liebherrstrasse
20, D-8000 München 26 (DE)**

## Beschreibung

Die Erfindung bezieht sich auf eine femorale Hüftgelenkendoprothese mit einem in die Markhöhle des Oberschenkelknochens einzusetzenden Schaft und einer den Schaft proximal abschließenden Auflageplatte, deren Unterfläche zur Auflage auf einer den Hals vom Oberschenkelknchen abtrennenden Resektionsfläche bestimmt ist und zum Zusammenwirken mit formentsprechend vorbearbeiteten Teilflächen der Resektionsfläche aus Teilflächen unterschiedlicher Neigung zusammengesetzt ist, die im medialen Teil der Unterfläche einen größeren Winkel mit der Richtung des Oberschenkelknochens einschließen als im lateralen Teil.

Bei den meisten bekannten Hüftgelenkendoprothesen ist die Unterfläche der Auflageplatte eben. Da sie schräg zur Hauptbelastungsrichtung liegt, ist ihre Fähigkeit zur Kraftübertragung begrenzt. Überdies neigt sie aufgrund des Effekts der schiefen Ebene dazu, eine medial gerichtete Relativbewegung der Prothese gegenüber dem Knochen hervorzurufen. Um dies zu verhindern, hat man die Unterfläche der Auflageplatte auch schon mit Riefen oder Zähnen versehen, die beim Einsetzen der Prothese in die Knochenoberfläche eingepreßt werden und damit eine Verankerung gegen seitliche Relativbewegungen ergeben sollen (DE-A-2 839 093). In der Praxis läßt sich dieses Ziel allerdings nur begrenzt erreichen, weil es sehr schwer ist, die Prothese bei der Operation so stark gegen die Resektionsfläche zu pressen, daß die Zähne hinreichend in die harte Corticalis eindringen. Es ist auch schwierig, die Resektionsfläche so genau zu formen, daß bei vorschriftsmäßigem Sitz der Prothese die Unterfläche der Auflageplatte genau parallel zur Resektionsfläche liegt, so daß sämtliche Zähne am Eingriff gleichmäßig beteiligt werden können.

Es ist eine Hüftgelenkendoprothese bekannt (DE-A-2 931 750), bei welcher die durchgehend eben geformte Unterfläche der Auflageplatte weniger stark geneigt verlaufen soll, als dies sonst üblich ist. Jedoch erscheint die Realisierbarkeit dieses Vorschlags höchst fraglich, weil die Neigungsrichtung der Resektionsfläche nicht frei wählbar, sondern vorgegeben ist durch die Lage des großen und kleinen Trochanter, an denen sie proximal benachbart entlanggeführt werden muß, so daß sich bei durchgehend ebener Auflageplatte im allgemeinen ein Neigungswinkel von etwa 45° ergibt.

Ferner ist eine Hüftgelenkendoprothese bekannt (FR-A- 1 416 534), deren Auflagefläche sich aus einer nahezu parallel zum Prothesenschaft verlaufenden, lateralen Teilfläche und einer quer dazu verlaufenden medialen Teilfläche zusammensetzt, die von lateral nach medial ein wenig ansteigt. Dies hat den Nachteil, daß zur Aufnahme des die Auflagefläche bildenden, klotzartigen oberen Prothesenteils der Oberschenkelknochen großvolumig ausgeschnitten werden muß und dadurch stark geschwächt wird.

Schließlich ist eine Huftgelenkendoprothese bekannt ( US-A-3 228 393), bei welcher die Unterfläche der Auflageplatte aus zwei Teilflächen zusammengesetzt ist, von denen die mediale stark gegenüber der Knochenlängsrichtung in üblicher Weise geneigt ist, während die laterale gegensinnig winklig zur medialen Teilfläche derart angeordnet ist, daß sie von medial nach lateral abfällt. Den dachförmig konkav zueinander angeordneten Teilflächen entsprechen formgleich vorbearbeitete Teilflächen der Resektionsfläche. Dadurch soll ein guter Sitz der Prothese auf der Resektionsfäche erzielt werden. Jedoch hat diese Formgebung den Nachteil, daß an der äußeren Kante der lateralen Teilfläche ein spitzer Winkel entsteht, der zu starken Kerbwirkungen im empfindlichen Kraftübergangsbereich zum größeren Trochanter führt.

Der Erfindung liegt die Aufgabe zugrunde, eine Prothese der eingangs genannten Art zu schaffen, die eine sichere Kraftübertragung zwischen der Unterfläche der Auflageplatte und der Resektionsfläche des Oberschenkelknochens ermöglicht, ohne daß dabei gefährliche Kerbwirkungen erzeugt werden.

Die erfindungsgemäße Lösung besteht darin, daß der mediale Teil der Unterfläche der Auflageplatte sich konkav stufig aus einer Teilfläche mit kleinem Neigungswinkel gegenüber der Halsrichtung der Prothese und einer weiter medial gelegenen, einen größeren Winkel mit dieser Richtung einschließenden Teilfläche zusammensetzt.

Zwar ergeben die beiden Teiflächen dabei eine ähnliche Dachform wie bei der zuletzt erörterten bekannten Prothese; jedoch ist diese Dachform insgesamt mehr nach medial verschoben, so daß lateral Platz für eine einen sanften und ggf. geschwungenen übergang zum größeren Trochanter schaffende Teilfläche verbleibt.

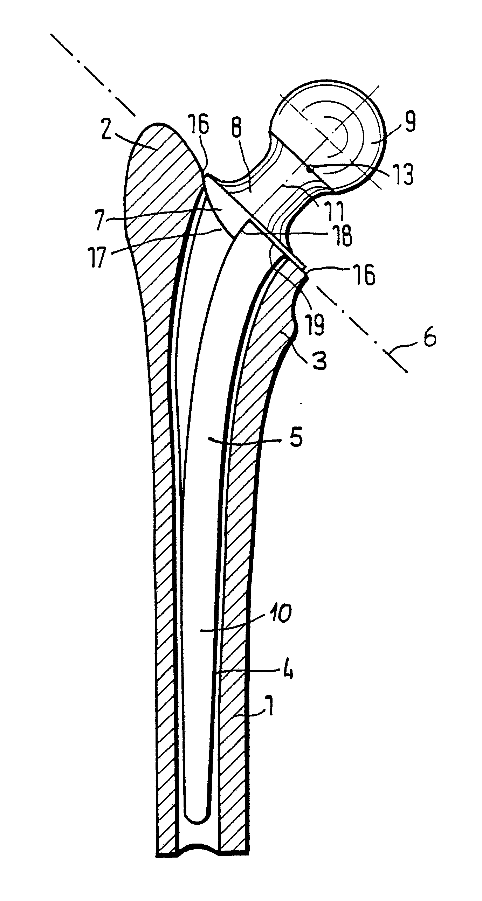

Ein vorteilhaftes Ausführungsbeispiel der Erfindung ist in der Zeichnung veranschaulicht. Darin zeigt die einzige Figur einen Längsschnitt durch den proximalen Bereich des Oberschenkelknochens mit eingesetzter Prothese.

In der Zeichnung erkennt man schraffiert die Schnittfläche des Knochens 1 mit großem Trochanter 2 und kleinem Trochanter 3 und Markhöhle 4, die zur Aufnahme des Prothesenschafts 5 vorbearbeitet ist. Der Oberschenkelhals ist längs der Ebene reseziert, deren Hauptrichtung durch die strichpunktierte Linie 6 angedeutet ist und die lateral so hoch liegt, daß der große Trochanter 2 erhalten bleibt, während sie medial so tief liegt, daß der Halsteil etwa die dargestellte Länge haben kann, die erforderlich ist, um dem Oberschenkelknochen hinreichende Schwenkbeweglichkeit gegenüber dem Acetabulum zu verleihen. Die Resektionslinie 6 verläuft daher etwa unter einem Winkel von 45° zur Längsrichtung des Oberschenkelknochens 1.

Die Prothese besteht aus dem Schaft 5, der Auflageplatte 7, dem Halsteil 8 und dem Gelenkkopf 9. Der Schaft ist mit oder ohne Knochenzement in die Markhöhle 4 eingesetzt und gibt in seinem distalen Abschnitt 10 die Richtung des Oberschenkelknochens an. Die Achse 11 des Halsteils 8 verläuft unter 45° zur Richtung des Oberschenkelknochens.

Die Unterfläche der Auflageplatte 7 und somit auch die Resektionsfläche des Oberschenkelknochens setzen sich aus einem gekrümmten, verhältnismäßig steil verlaufenden, lateralen Flächenanteil 17, aus einer etwa in der Mitte der Unterfläche in einer Ebene quer zur Zeichenebene parallel zur Achse 11 des Oberschenkelhalses 8 verlaufenden Stufenfläche 18 sowie einer lotrecht dazu verlaufenden Fläche 19 zusammen, wobei die Fläche 17 zylindrisch um die lotrecht zur Zeichenebene verlaufende Achse 13 geformt ist. Die Stufenfläche 18 nimmt diejenigen Kraftkomponenten auf, die aufgrund der Wirkung der Flächenanteile 17 und 19 als schiefe Ebene die Prothese relativ zum Knochen nach medial drängen. In ihrer Gesamtheit sind die Flächen 18 und 19 daher ausgezeichnet zur Aufnahme vertikaler Kräfte unter Vermeidung jeglicher medialen Verschiebung der Prothese gegenüber dem Knochen geeignet.

Da alle diese Flächen sich aus parallelen, lotrecht zur Zeichenebene verlaufenden Erzeugenden zusammensetzen, läßt sich die Resektionsfläche 16 des Knochens hinreichend leicht und genau formen.

Es ist nicht erforderlich, daß die Fläche 18 so steil, d.h. in so kleinem Winkel zur Halsrichtung verläuft, wie dies in der Zeichnung dargestellt ist. Oftmals genügt ein flacherer Winkel, wobei dann auch der Flächenanteil 19 entsprechend flacher ausgeführt werden kann. Der laterale, steiler verlaufende Flächenanteil 17 ist vorteilhafterweise eine von der ebenen Gestalt abweichende Rotationsfläche.

## Patentansprüche

Femorale Hüftgelenkendoprothese mit einem in die Markhöhle (4) des Oberschenkelknochens (1) einzusetzenden Schaft (5) und einer den Schaft proximal abschließenden Auflageplatte (7), deren Unterfläche zur Auflage auf einer den Hals vom Oberschenkelknochen abtrennenden Resektionsfläche (16) des Knochens bestimmt ist und zum Zusammenwirken mit formentsprechend vorbearbeiteten Teilflächen der Resektionsfläche (16) aus Teilflächen unterschiedlicher Neigung zusammengesetzt ist, die im medialen Teil der Unterfläche einen größeren Winkel mit der Richtung des Oberschenkelknochens einschließen als im lateralen Teil, dadurch gekennzeichnet, daß der mediale Teil der Unterfläche der Auflageplatte (7) sich konkav stufig aus einer Teilfläche (18) mit kleinem Neigungswinkel gegenüber der Halsrichtung (11) der Prothese und einer weiter medial gelegenenen, einen größeren Winkel mit dieser Richtung einschließenden Teilfläche (19) zusammensetzt.

## Claim

A femoral hip-joint endoprosthesis with a shaft (5) to be inserted in the medullary cavity (4) of the femur (1) and with a bearing plate (7) terminating the shaft at its proximal end, the undersurface (21) of which bearing plate is intended to bear against a resection surface (16) of the bone separating the neck from the femur, and has surface portions of different inclination for cooperation with surface portions of the resection surface (16), which surface portions have been previously prepared so as to be of matching shape and which in the medial portion of the undersurface form a larger angle with the direction of the femur than in the lateral portion, characterised in that the medial portion of the undersurface of the bearing plate (7) is composed, in concave stepped manner, of a surface portion (18) with a small angle of inclination relative to the neck direction (11) of the prosthesis and of a further medially situated surface portion (19) forming a larger angle with this direction.

## Revendication

Prothèse fémorale de hanche, comprenant une tige (5) destinée à être insérée dans la cavité médullaire (4) du fémur (1) et une plaque d'appui (7) qui termine la tige à son extrémité praximale et dont la surface inférieure est destinée à reposer sur une surface de resection (16) de l'os, séparant le col du carps du fémur, et est composée, paur coopérer avec des surfaces partielles de la surface de résection (16), préparées à la forme adéquate, de surfaces partielles ayant des inclinaisans différentes qui forment, avec la direction du corps du fémur, un angle plus grand dans la partie interne de la surface inférieure que dans la partie externe, caractérisée en ce que la partie interne de la surface inférieure de la plaque d'appui (7) se compose, en gradins cancaves, d'une surface partielle (18) présentant un petit angle d'inclinaisan par rapport à la direction (11) du col de la prathèse et d'une surface partielle (19) plus interne formant un angle plus grand avec cette direction.